(19) **Europäisches Patentamt**

**European Patent Office**

**Office européen des brevets**

(11) Publication number: **0 292 395 B1**

(12) **EUROPEAN PATENT SPECIFICATION**

(45) Date of publication of patent specification: **08.12.93**   (51) Int. Cl.⁵: **A61M 1/36**

(21) Application number: **88401221.2**

(22) Date of filing: **19.05.88**

(54) **Blood reservoir for an extracorporeal blood treatment circuit.**

(30) Priority: **19.05.87 JP 122243/87**

(43) Date of publication of application:
**23.11.88 Bulletin 88/47**

(45) Publication of the grant of the patent:
**08.12.93 Bulletin 93/49**

(84) Designated Contracting States:
**BE DE FR GB IT SE**

(56) References cited:
**EP-A- 0 146 708**
**US-A- 4 620 965**

(73) Proprietor: **TERUMO KABUSHIKI KAISHA**
**No. 44-1, Hatagaya 2-chome,**
**Shibuya-ku**
**Tokyo 151(JP)**

(72) Inventor: **Katsura, Yoshiro**
**c/o Terumo Kabushiki Kaisha**
**2656-1, Ohbuchi**
**Fuji-shi Shizuoka-ken(JP)**

(74) Representative: **Joly, Jean-Jacques et al**
**Cabinet Beau de Loménie**
**158, rue de l'Université**
**F-75340 Paris Cédex 07 (FR)**

**Description**

BACKGROUND OF THE INVENTION

The present invention relates to a blood reservoir for temporarily storing blood in a extracorporeal blood circulating circuit.

When a thoracic operation, for example, is to be carried out on a patient, an extracorporeal blood circulating circuit is established using an artificial lung in which the blood is circulated for an exchange of carbon dioxide an oxygen. The blood delivered from the artificial lung is temporarily stored in a blood reservoir for removal of air bubbles from the blood and a steady supply of the blood. Thereafter, the blood is pumped into the patient under the operation at a constant pulse rate.

Blood reservoirs for use in extracorporeal blood circulation include a closed-type blood reservoir in the form of a soft bag for storing blood in a airtight condition and an open-type blood reservoir in the form of a hard housing for storing blood. The open-type blood reservoir is advantageous in that priming and confirmation of the stored amount of blood can easily be performed, and it would be easy to construct the blood reservoir as a unitary component of an artificial lung. An artificial lung with centralized functions, employing an open-type blood reservoir has been proposed by the applicant (see patent document US-A-4 620 965.)).

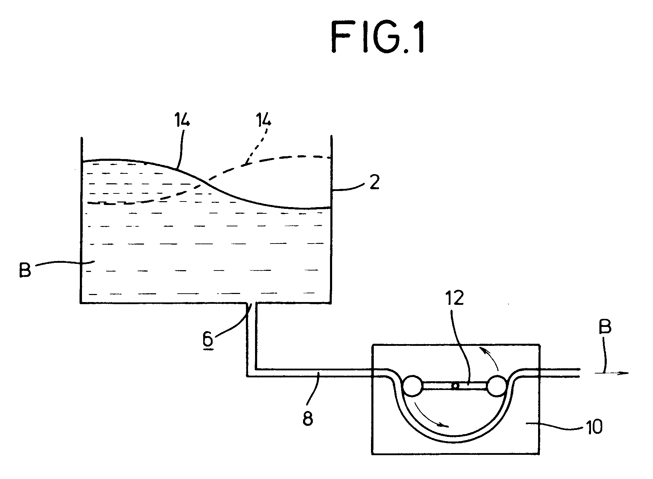

FIG. 1 of the accompanying drawings schematically illustrates a extracorporeal blood circulating circuit employing an open-type blood reservoir. A blood reservoir 2 stores blood B supplied from an artificial lung (not shown). The blood B is delivered out of the blood reservoir 2 from a blood discharge port 6 defined in the bottom of the tank 2 and through a flexible pipe 8. The flexible pipe 8 extends through a pump 10 which includes a rotor 12 that rotates in the direction of the arrows for pulsatively feeding the blood B through the flexible pipe 8 at a certain pulse rate.

The surface level 14 of the blood B stored in the blood reservoir 2 may be subjected to resonant vibration as indicated by the solid and broken lines due to various conditions or parameters such as the pulse rate of the pump 10, i.e., the speed of rotation of the rotor 12, the amount of the blood or the area of the surface level of the blood stored in the blood reservoir 2, and the viscosity of the blood B. When the blood B undergoes such resonant vibration, unwanted vibration tends to be applied to the artificial lung and other components of the extracorporeal blood circulating circuit. The amplitude of the resonant vibration is particularly large if the blood discharge port 6 is positioned asymmetrically with respect to the surface level 14. To avoid this shortcoming, the blood discharge port 6 should preferably be located substantially centrally in the blood reservoir 2. With the blood discharge port 6 thus positioned, however, the configuration and other design factors of the blood reservoir 2 are greatly limited.

SUMMARY OF THE INVENTION

It is a primary object of the present invention to provide a blood reservoir for temporarily storing blood in a extracorporeal blood circulating circuit, wherein resonant vibrations at the blood surface level are substantially eliminated.

According to the invention, the blood reservoir includes a substantially vertical partition which is effect for suppressing resonant vibration of the surface level of stored blood which would otherwise be caused by pulsatory blood discharge. Thereby, unwanted vibration will not be applied to the extracorporeal blood circulating circuit, and an artificial lung and other components coupled to the blood reservoir can be arranged with greater freedom.

The blood discharge port defined in a bottom of the blood storage section for discharging the blood, is positioned asymmetrically with respect to the blood storage section.

The partition may comprise a plurality of substantially vertical plates which extend in perpendicular relation to each other, or which substantially divide the blood storage section into a plurality of regions.

The blood storage section may be of a tapered configuration with its volume progressively smaller toward the blood discharge port, the partition being disposed on a slanted surface of the tapered blood storage section.

The partition has a height which is preferably at least 80 % of the height of the surface level of the stored blod which would be subjected to resonant vibration if the partition were dispensed with.

The partition may be designed to divide the blood storage section with a gap being left between the partition and an inner wall surface of the blood storage section.

The partition substantially divides 30 % to 90 %, preferably about 60 %, of the blood storage section.

The above and other features and advantages of the present invention will become more apparent from the following description when taken in conjunction with the accompanying drawings in which preferred embodiments of the present invention are shown by way of illustrative example.

BRIEF DESCRIPTION OF THE DRAWINGS

FIG. 1 is a schematic elevational view of a extracorporeal blood circulating circuit employing a blood reservoir of the prior art shown as a comparative example ;
FIG. 2 is a schematic elevational view, partly in cross section, of a blood reservoir according to the present invention, coupled to an artificial lung ;
FIG. 3 is a schematic perspective view of the blood reservoir illustrated in FIG. 2 ;
FIG. 4 is a schematic perspective view of the blood reservoir shown in FIGS. 2 and 3 ;
FIG. 5 is a schematic view of a blood circulation experimenting circuit for showing the characteristics of the blood reservoir of the invention ; and
FIGS. 6(a) through 6(d) are schematic views of blood reservoirs according to other embodiments of the present invention.

DETAILED DESCRIPTION OF THE PREFERRED EMBODIMENTS

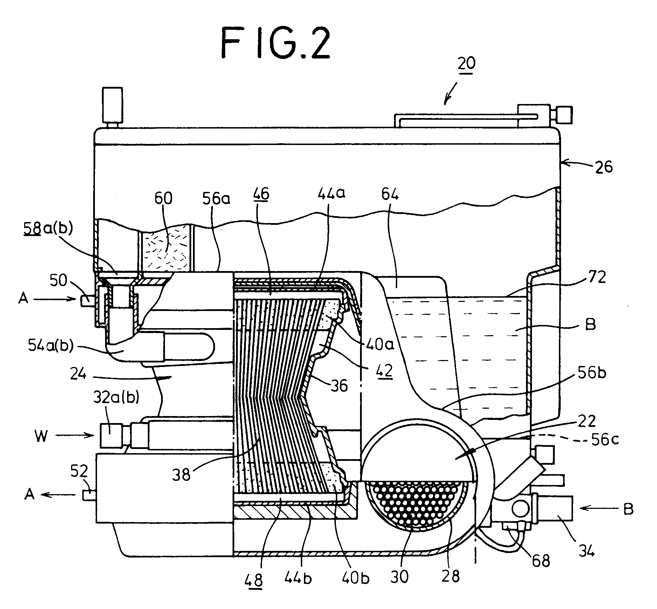

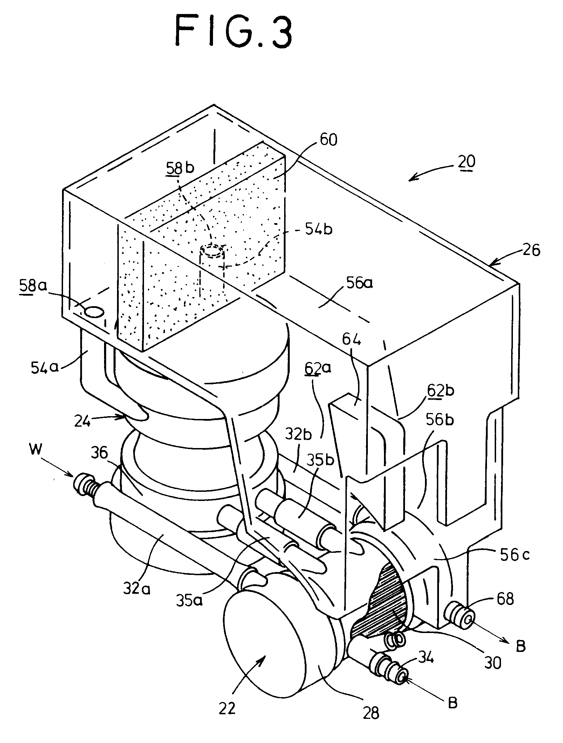

As shown in FIGS. 2 and 3, an extracorporeal blood circulating apparatus has a unitary apparatus assembly 20 comprising a heat exchanger 22, an artificial lung 24, and an open-type blood reservoir 26 according to the present invention.

The heat exchanger 22 includes a tubular housing 28 accommodating therein a number of heat exchanger pipes 30. Warm or cold water W is supplied from a water port 32a to the pipes 30, and then discharged from the pipes 30 via a water port 32b. Blood B flowing from a blood supply port 34 into the heat exchanger 22 is heated or cooled to a prescribed temperature while it is passing around the pipes 30. Thereafter, the blood B is supplied via two joint tubes 35a, 35b (see FIG. 3) to the artificial lung 24.

The artificial lung 24 serves to remove carbon dioxide from the blood B and add oxygen to the blood B. The artificial lung 24 includes a multiplicity of hollow filamentary membranes 38 blundled and stored in a substantially cylindrical housing 36 with a constricted central portion. On the upper and lower ends of the housing 36, there are mounted partitions 40a, 40b holding the opposite ends of the hollow filamentary membranes 38. The housing 36 and the partitions 40a, 40b jointly define a space surrounded thereby and housing the hollow filamentary membranes 38 therein, the space communicating with the heat exchanger 22. This space serves as a gas exchanging chamber 42 through which the blood B flows. Covers 44a, 44b are also mounted on the upper and lower ends of the housing 36. The partition 40a and the cover 44a define a gas supply chamber 46 therebetween, and the partition 40b and the cover 44b define a gas discharge chamber 48 therebetween. The hollow filamentary membranes 38 are supplied with a gas A containing oxygen from a gas inlet port 50 via the gas supply chamber 46. The gas A delivered from the hollow filamentary membranes 38 is discharged through the gas discharge chamber 48 from a gas outlet port 52.

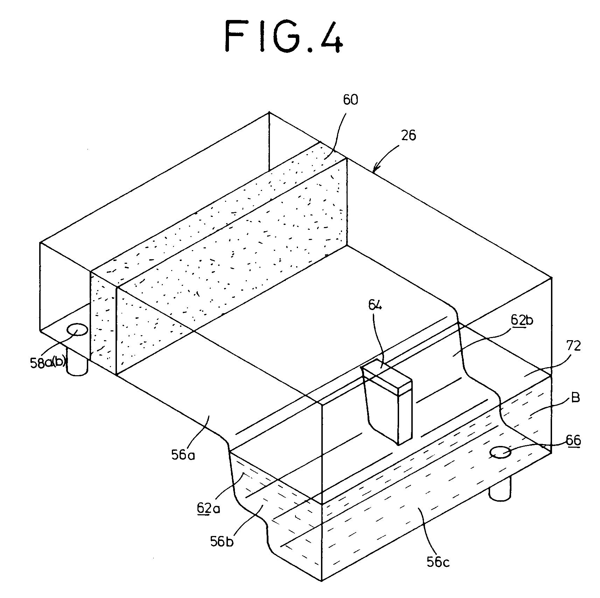

The blood reservoir 26 is held in communication with the gas exchanging chamber 42 of the artificial lung 24 through joint tubes 54a, 54b. As schematically shown in FIG. 4, the blood reservoir 26 has a bottom plate composed of first through third steps 56a, 56b, 56c. The joint tubes 54a, 54b have blood inlet ports 58a, 58b, respectively, opening at the first uppermost step 56a. A urethane anti-foaming member 60 is disposed on the first step 56a near the blood inlet ports 58a, 58b for preventing the blood B as it flows in from being foamed. A partition 64 is mounted substantilly centrally on the second intermediate step 56b and divides the blood storage space on the second step 56b into a first blood storage region 62a and a second blood storage region 62b. A blood discharge port 66 for discharging the blood B stored in the blood reservoir 26 opens at the end of the third lowermost step 56c. In operation, the blood discharge port 66 communicates with a pump (not shown) which delivers the blood at a prescribed pulse rate through a blood outlet port 68. In the case, the pump may be a rotary type a peristaltic finger type.

The blood reservoir of the invention is basically of the above structure. Now, operation and advantages of the extracorporeal blood circulating apparatus will be described below.

The blood B coming from the blood supply port 34 is supplied through the gaps between the tubes 30 stored in the heat exchanger 22 to the artificial lung 24. At this time, water W maintained at a prescribed temperature is flowing through the heat exchanger tubes 30 through the water ports 32a, 32b. The blood B is heated or cooled to a predetermined temperature by the water W flowing through the tubes 30.

EP 0 292 395 B1

The blood B fed from the heat exchanger 22 into the artificial lung 24 enters the gas exchanging chamber 42 surrounded by the housing 36 in which oxygen is added to the blood B and carbon dioxide is removed from the blood B. More specifically, the gas A containing oxygen is supplied into the bundled hollow filamentary membranes 38 in the housing 36 from the gas inlet port 50 through the gas supply chamber 46. Oxygen and carbon dioxyde are exchanged in the blood B through the hollow filamentary membranes 38. The gas A containing carbon dioxide is discharged via the gas discharge chamber 48 from the gas outlet port 66.

The blood B to which oxygen has been added in the artificial lung 24 then flows through the joint tubes 54a, 54b and the blood inlet ports 58a, 58b into the blood reservoir 26. After air bubbles of foams contained in the blood B have been removed by the anti-foaming member 60, the blood B is stored in the blood reservoir 26.

The blood B stored in the blood reservoir 26 is then intermittently or pulsatively delivered out through the blood discharge port 66 by the pump (not shown) coupled to the blood outlet port 68. The surface level 72 of the blood B stored in the blood reservoir 26 would be subjected to resonant vibration due to intermittent delivery of the blood B by the pump. However, such resonant vibration is highly effectively suppressed by the partition 64 (which divides 60 % of the blood storage section in the illustrated embodiment) on the second step 56b in the blood reservoir 26.

The effects of the partition 64 will be described in detail based on experimental results.

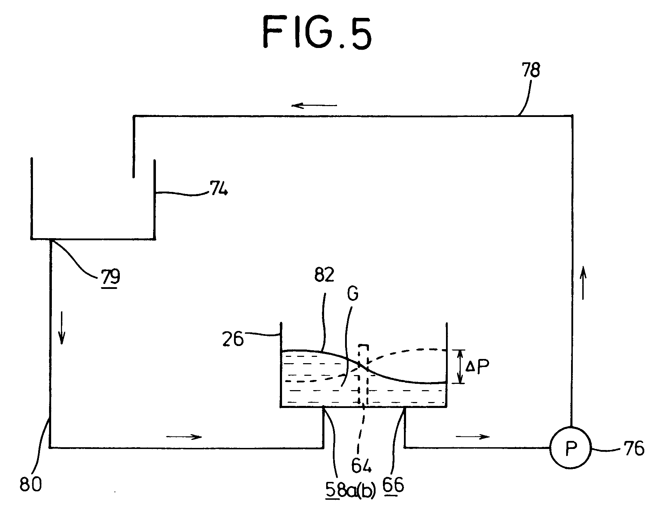

FIG. 5 schematically shows a blood circulation experimenting circuit employing the blood reservoir 26. A water storage tank 74 is disposed in a space above the blood reservoir 26. The blood discharge port 66 of the blood reservoir 26 is connected to the water storage tank 74 by a tube 78 through a pump 76 which can pulsatively feed water. The blood inlet port 58a (58b) of the blood reservoir 26 is connected to a discharge port 79 of the water storage tank 74 by means of a tube 80. Each of the blood reservoir 26 and the water storage tank 74 stores an aqueous solution G of 35 % of glycerin having substantially the same property (a viscosity of about 3 centipoises) as that of blood B.

With the partition 64 removed from the blood reservoir 26, the aqueous solution G of glycerin was circulated by the pump 76 at a pulse rate of 100 beats/min. The amount of change or fluctuation P of the surface level 82 due to the pulsative blood feed was varied with respect to the amount of water stored in the blood reservoir 26 as indicated by the following Table 1 :

4

Table 1

| Amount of stored water (ml) | Without partition P (mm) | With partition P (mm) |
|---|---|---|
| 1200 | 100 | 100 |
| 1100 | 150 | 100 |
| 1000 | 150 | 100 |
| 900 | 250 | 120 |
| 800 | 300 | 50 |
| 750 | 350 | 50 |
| 700 | 300 | 50 |
| 600 | 100 | 50 |

When the amount of water stored in the blood reservoir 26 was in the range of from 700 to 800 ml, the fluctuation P of the surface level 82 was maximum, and the generation of resonant vibration was observed.

When the partition 64 was substantially vertically mounted on the substantially central area of the second step 56b in the blood reservoir 26, as shown in FIG. 4, the fluctuation P of the surface level 82 was greatly reduced and no resonant vibration was produced, as indicated by the Table 1.

More specifically, it is considered that resonant vibration which would otherwise be caused on the surface level 72 (see FIGS. 2 and 4) of the blood B stored in the blood reservoir 26 would be produced when the natural frequency of the blood reservoir system based on the characteristics of the blood B, the configuration of the blood reservoir 26, and other parameters coincides with the pulse rate of the pump. The pulse rate of the pump is selected to be in a certain range for practical reasons with respect to the artificial lung 24. The partition 64 located in the blood reservoir 26 is effective in making the system natural frequency widely different from the pulse rate of the pump, thus suppressing the undesirable resonant vibration. Therefore, when the pump is actuated, the surface level 72 of the blood B is kept calm without violent turbulent motion. Since the fluctuation P of the surface level 72 arising from the pulse rate of the pump is small, the height of the surface level 72, i.e., the amount of the blood B stored in the blood reservoir 26 can easily and accurately be checked by a visual inspection. Moreover, inasmuch as any turbulent motion of the blood B in the blood reservoir 26, is effectively suppressed, vibration of the blood B is held to a minimum, and no unwanted vibration is applied to the apparatus. Since the interior space of the blood reservoir 26 is divided by the partition 64, it is not necessary to position the blood discharge port 66 centrally in the bottom of the blood reservoir 26. Accordingly, the artificial lung 24 and other components which are coupled to the blood reservoir 26 can be arranged with greater freedom.

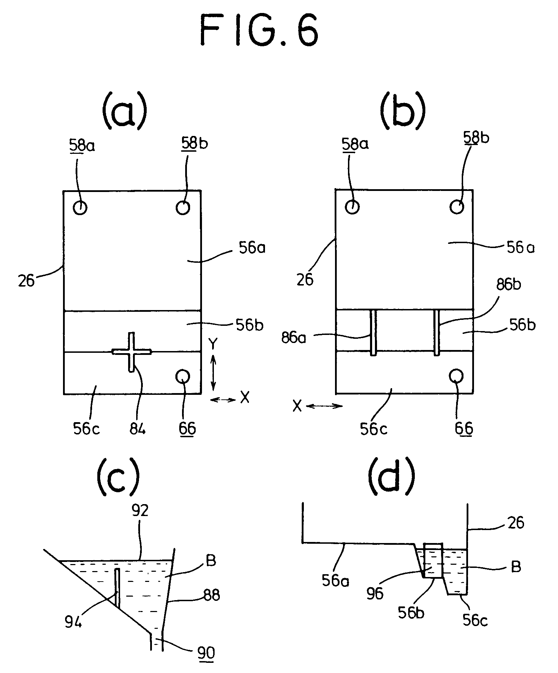

The blood reservoir 26 and the partition 64 are not limited to the above configurations, but may be of various shapes. For example, FIG. 6(a) shows a crisscross partition 84 mounted in the blood reservoir 26 for suppressing vibration of the blood B in X and Y directions. FIG. 6(b) illustrates two parallel partitions 86a, 86b mounted in the blood reservoir 26 and spaced in a X direction for particularly suppressing vibration in

5

the X direction. FIG. 6(c) shows a blood reservoir 88 in the form of an inverted circular cone or an inverted pyramid, with a partition 94 disposed on a slanted surface in the blood reservoir 88. The position of a blood discharge port 90 of the tank 88 is substantially at the center of the bottom of the blood storage region surrounded by the partition 94 and the righthand wall (as shown) of the tank 88 for effectively suppressing vibration of the blood B. FIG. 6(d) shows a partition 96 mounted on the second step 56b of the blood reservoir 26 shown in FIGS. 2 through 4 and held out of contact with the wall of the tank 26 at the second step 56b for reducing vibration of the blood B.

The partition 64 shown in FIGS. 2 and 3 is not limited to a certain cross-sectional shape insofar as it can sufficiently withstand the pressure of the blood stored in the blood reservoir 26. The partition 64 may be integrally formed with the blood reservoir 26. Therefore, the partition 64 can easily be manufactured. The height of the partition 64 should be at least 80 % of the height of the surface level 72 which would be subjected to resonant vibration if the partition 64 were dispensed with. The partition 64 should be of as wide an area as possible for effective suppression of vibration of the blood B. The partition 64 should be of such a size as to divide 30 % to 90 % of the blood storage section in the blood reservoir 26. If the partition 64 divides 30 % or more of the blood storage section, it can sufficiently suppress vibration of the blood. If the partition 64 divides 90 % or less of the blood storage section, and where the blood discharge port is defined in one of the divided areas of the blood storage section, the blood can flow sufficiently between the divided areas, and hence it is not necessary to add extra blood to one of the divided areas which has the blood discharge port in order to compensate for a blood shortage in that divided area. Moreover, the possibility of resonant vibration in that divided area only is eliminated.

With the present invention, as described above, the blood reservoir for temporarily storing blood in the extracorporeal blood circulating apparatus intermittently delivers out the stored blood through the blood discharge port defined in the bottom of the tank, and the blood reservoir has a substantially vertical partition disposed therein. The partition is effective in making the system natural frequency, which is determined by the shape of the blood reservoir and the properties of the blood, widely different from the pulse rate at which the blood is delivered from the blood reservoir. Therefore, the surface level of the stored blood is free from resonant vibration. As a result, unwanted vibration is prevented from being applied to the extracorporeal blood circulating circuit. Since the blood discharge port of the blood reservoir can be positioned considerably freely, the artificial lung and other components coupled to the blood reservoir can also be arranged with greater freedom.

The present invention is not limited to the illustrated embodiment, but the principles of the invention are also applicable to a extracorporeal blood circulating apparatus which has no pump, or a blood reservoir which is separate from an artificial lung or a heat exchanger, for example.

## Claims

1. A blood reservoir (26) comprising a blood storage section for temporarily storing blood, and a blood discharge port (66) defined in a bottom of said blood storage section for discharging the blood, said blood discharge port being positioned asymmetrically with respect to said blood storage section, characterized in that at least one partition (64 ; 84 ; 86a-86b ; 94 ; 96) is disposed substantially vertically in said blood storage section for suppressing resonant vibration of a surface level of the stored blood upon pulsative blood discharge from the blood storage section.

2. A blood reservoir according to claim 1, characterized in that said partition (84) comprises a plurality of substantially vertical plates extending in perpendicular relation to each other.

3. A blood reservoir according to claim 1, characterized in that said partition comprises a plurality of substantially vertical plates (86a, 86b) which substantially divide said blood storage section into a plurality of regions.

4. A blood reservoir according to claim 1, 2 ou 3, characterized in that said blood storage section (88) is of a tapered configuration with its cross-sectional area becoming progressively smaller toward said blood discharge port, said partition (94) being disposed on a slanted surface of the tapered blood storage section.

5. A blood reservoir according to claim 1, characterized in that said partition has a height which is at least 80 % of the height of the surface level of the stored blood which would be subjected to resonant vibration if the partition were dispensed with.

6. A blood reservoir according to claim 1, characterized in that said partition divides the blood storage section with a gap being left between the partition and an inner wall surface of said blood storage section.

7. A blood reservoir according to claim 6, characterized in that said partition substantially divides 30 % to 90 % of said blood storage section.

8. A blood reservoir according to claim 7, characterized in that said partition substantially divides 60 % of said blood storage section.

**Patentansprüche**

1. Blutbehälter (26) mit einem Blutspeicherabschnitt zum vorübergehenden Speichern von Blut und einem zur Ausgabe des Blutes in einem Unterteil des Blutspeicherabschnitts gebildeten Blutauslaß (66), wobei der Blutauslaß asymmetrisch zum Blutspeicherabschnitt angeordnet ist, dadurch gekennzeichnet, daß mindestens eine Trennwand (64; 84; 86a-86b; 94; 96) im wesentlichen vertikal im Blutspeicherabschnitt vorgesehen ist, um bei pulsierender Blutausgabe aus dem Blutspeicherabschnitt eine Resonanzschwingung eines Flüssigkeitsspiegels des gespeicherten Blutes zu unterdrücken.

2. Blutbehälter nach Anspruch 1, dadurch gekennzeichnet, daß die Trennwand (84) eine Mehrzahl von im wesentlichen vertikalen Platten aufweist, welche sich in senkrechter Beziehung zueinander erstrecken.

3. Blutbehälter nach Anspruch 1, dadurch gekennzeichnet, daß die Trennwand eine Mehrzahl von im wesentlichen vertikalen Platten (86a, 86b) aufweist, welche den Blutspeicherabschnitt im wesentlichen in eine Mehrzahl von Bereichen teilen.

4. Blutbehälter nach Anspruch 1, 2 oder 3, dadurch gekennzeichnet, daß der Blutspeicherabschnitt (88) eine sich verengende Konfiguration aufweist, wobei seine Querschnittfläche auf den Blutauslaß hin zunehmend kleiner wird, und die Trennwand (94) auf einer geneigten Oberfläche des sich verengenden Blutspeicherabschnitts angeordnet ist.

5. Blutbehälter nach Anspruch 1, dadurch gekennzeichnet, daß die Trennwand eine Höhe aufweist, welche vorzugsweise mindestens 80% der Höhe des Flüssigkeitsspiegels des gespeicherten Blutes beträgt, welcher ohne die Trennwand eine resonante Vibration erfahren würde.

6. Blutbehälter nach Anspruch 1, dadurch gekennzeichnet, daß die Trennwand den Blutspeicherabschnitt unterteilt, wobei ein Spalt zwischen der Trennwand und einer Innenwandoberfläche des Blutspeicherabschnitts gelassen ist.

7. Blutbehälter nach Anspruch 6, dadurch gekennzeichnet, daß die Trennwand im wesentlichen 30 bis 90% des Blutspeicherbereichs abtrennt.

8. Blutbehälter nach Anspruch 7, dadurch gekennzeichnet, daß die Trennwand im wesentlichen 60% des Blutspeicherbereichs abtrennt.

**Revendications**

1. Réservoir de sang (26) comprenant une partie de stockage du sang destinée au stockage temporaire du sang et un orifice d'évacuation du sang (66) défini dans un fond de ladite partie de stockage du sang et destiné à l'évacuation du sang, ledit orifice d'évacuation du sang étant positionné de façon asymétrique par rapport à ladite partie de stockage du sang, caractérisé en ce qu'au moins une cloison (64 ; 84 ; 86a-86b ; 94 ; 96) est disposée de façon sensiblement verticale dans ladite partie de stockage du sang pour supprimer la vibration résonante au niveau de la surface du sang stocké lors de l'évacuation pulsatoire du sang de la partie de stockage du sang.

2. Réservoir de sang selon la revendication 1, caractérisé en ce que ladite cloison (84) comprend une pluralité de plaques sensiblement verticales s'étendant perpendiculairement les unes aux autres.

**3.** Réservoir de sang selon la revendication 1, caractérisé en ce que ladite cloison comprend une pluralité de plaques sensiblement verticales (86a, 86b) qui divisent sensiblement ladite partie de stockage du sang en une pluralité de régions.

**4.** Réservoir de sang selon la revendication 1,2 ou 3, caractérisé en ce que ladite partie de stockage du sang (88) a une configuration en biseau, son aire transversale diminuant progressivement en direction dudit orifice d'évacuation du sang, ladite cloison (94) étant disposée sur une surface inclinée de la partie oblique de stockage du sang.

**5.** Réservoir de sang selon la revendication 1, caractérisé en ce que ladite cloison a une hauteur qui est au moins égale à 80% de la hauteur de la surface du sang stocké qui serait soumise à une vibration résonante si on supprimait la cloison.

**6.** Réservoir de sang selon la revendication 1, caractérisé en ce que ladite cloison sépare la partie de stockage du sang, un espace demeurant entre la cloison et une surface pariétale interne de ladite partie de stockage du sang.

**7.** Réservoir de sang selon la revendication 6, caractérisé en ce que ladite cloison sépare sensiblement 30% à 90% de ladite partie de stockage du sang.

**8.** Réservoir de sang selon la revendication 7, caractérisé en ce que ladite cloison sépare sensiblement 60% de ladite partie de stockage du sang.

# FIG.1

EP 0 292 395 B1

# FIG.2

EP 0 292 395 B1

# FIG.3

# FIG.4

# FIG.5

EP 0 292 395 B1

# FIG.6

## (a)

58a 58b

56a

26

56b

Y
↕

56c 84 66

X ↔

## (b)

58a 58b

26 56a

86b

86a 56b

X ↔ 56c 66

## (c)

92

B

88

94

90

## (d)

26

B

56a 96 56b 56c